# EUROPEAN PATENT APPLICATION

(11) **EP 3 279 578 A1**
(43) Date of publication of application: **07.02.2018**
(21) Application number: 15887532.8
(22) Date of filing: 30.03.2015
(51) Int. Cl.: F24F 11/02

(54) **AIR CONDITIONER, SENSOR UNIT AND AIR CONDITIONING SYSTEM**

(71) Applicant: Fujitsu Limited, Kawasaki-shi, Kanagawa 211-8588 (JP)
(72) Inventor: YAMAJI, Takayuki, Kawasaki-shi Kanagawa 211-8588 (JP)
(74) Representative: Hoffmann Eitle
(86) International application number: PCT/JP2015/059980
(87) International publication number: WO 2016/157379

(57) **Abstract**

An air conditioner 2 may include a sensor unit 5 that includes a Doppler sensor and an inertial sensor. Detection accuracy of biological information in a space corresponding to an air conditioning target of the air conditioner 2 can be improved.

## Description

### Technical Field

A technology discussed in the specification relates to an air conditioner, a sensor unit, and an air conditioning system.

### Background Art

Technologies for measuring biological information such as heartbeat, respiration, and motion of a living body have been studied and examined. For example, cooperation technologies between the measured biological information and an air conditioning control have also been examined.

### PRIOR ART DOCUMENTS

### Patent Documents

[Patent Document 1] Japanese Laid-open Patent Publication No. 2011-15887
[Patent Document 2] Japanese Laid-open Patent Publication No. 2013-24466
[Patent Document 3] Japanese Laid-open Patent Publication No. 2015-21658
[Patent Document 4] International Publication Pamphlet No. WO 2006/038441
[Patent Document 5] Japanese Laid-open Patent Publication No. 2014-39666
[Patent Document 6] Japanese Laid-open Patent Publication No. 2008-146866
[Patent Document 7] Japanese Laid-open Patent Publication No. 2014-058254

### SUMMARY OF THE INVENTION

### PROBLEM TO BE SOLVED BY THE INVENTION

Heartbeat, respiration, and motion of a living body can be detected using a Doppler sensor. However, in a space corresponding to an air conditioning target of the air conditioner, a noise component may be included in a detected value of the Doppler sensor due to a vibration generated during an operation of the air conditioner. For that reason, an error may occur in the detection of the biological information.

In one aspect, one object of a technology described in the specification is to improve a detection accuracy of biological information in a space corresponding to an air conditioning target of the air conditioner.

### MEANS TO SOLVE THE PROBLEM

In one aspect, an air conditioner may include a sensor unit including a Doppler sensor and an inertial sensor.

Further, in one aspect, the sensor unit may be a sensor unit attached to an air conditioner and may include a Doppler sensor and an inertial sensor. The sensor unit may be attached to a position receiving a vibration during an operation of the air conditioner.

Furthermore, in one aspect, an air conditioning system may include a sensor unit including a Doppler sensor and an inertial sensor, an air conditioner having the sensor unit attached thereto, and a control system. The control system may be connected to the sensor unit and the air conditioner to communicate therewith, may correct a detected value of the Doppler sensor received from the sensor unit in response to a detected value of the inertial sensor received from the sensor unit, and may transmit a signal generated based on a corrected value to the air conditioner.

### Effects of Invention

As one aspect, it is possible to improve the detection accuracy of the biological information in the space corresponding to an air conditioning target of the air conditioner.

### BRIEF DESCRIPTION OF DRAWINGS

Fig. 1 is a block diagram illustrating a configuration example of an air conditioning system according to one embodiment.
Fig. 2 is a block diagram illustrating a configuration example of a non-contact sleep sensor used in the air conditioning system illustrated in Fig. 1.
Fig. 3 is a block diagram illustrating a configuration example of the non-contact sleep sensor used in the air conditioning system illustrated in Fig. 1.
Fig. 4 is a block diagram illustrating a configuration example of the air conditioning system by focusing on a configuration of an air conditioner illustrated in Fig. 1.
Fig. 5 is a block diagram illustrating a configuration example of a control system used in the air conditioning system illustrated in Fig. 1.
Fig. 6 is a diagram schematically illustrating an example of an appearance of the non-contact sleep sensor used in the air conditioning system illustrated in Fig. 1.
Fig. 7 is a diagram illustrating an example of an attachment position of the non-contact sleep sensor with respect to the air conditioner illustrated in Fig. 1.
Fig. 8 is a diagram illustrating an example of an attachment position of the non-contact sleep sensor with respect to the air conditioner illustrated in Fig. 1.
Fig. 9 is a diagram illustrating a state where the non-contact sleep sensor is attached to an attachment jig of the air conditioner illustrated in Fig. 1.
Fig. 10 is a diagram illustrating an example of a change in output signal with time of a Doppler sensor and an inertial sensor of the non-contact sleep sensor illustrated in Figs. 1 to 3.
Fig. 11 is a diagram illustrating an example of a change in inertial sensor value with time and a change in body motion amount with time obtained based on an output signal of the Doppler sensor of the non-contact sleep sensor illustrated in Figs. 2 and 3.
Fig. 12 is a diagram illustrating an example (before correction) of a change in time of a calculation value (a determination value) for a sleep determination obtained based on the body motion amount illustrated in Fig. 11.
Fig. 13 is a diagram illustrating an example of a reference of the calculation value (the determination value) for the sleep determination.
Fig. 14 is a diagram illustrating an example (after correction) of a change in time of the calculation value (the determination value) for the sleep determination obtained based on the body motion amount illustrated in Fig. 11.
Fig. 15 is a diagram schematically illustrating a concept of an extended wavelength according to one embodiment.
Fig. 16 is a diagram illustrating a calculation example of the extended wavelength according to one embodiment.
Fig. 17 is a flowchart illustrating an operation example of the non-contact sleep sensor illustrated in Figs. 1 to 3.
Fig. 18 is a diagram illustrating an example of a change in time of the body motion amount before and after the correction using the output signal of the inertial sensor in the operation example of Fig. 17.
Fig. 19 is a flowchart illustrating a first modified example of Fig. 17.
Fig. 20 is a diagram illustrating an example of a correction of an extended wavelength of the first modified example.
Fig. 21 is a flowchart illustrating a second modified example of Fig. 17.
Fig. 22 is a diagram illustrating an example of a correction of a determination threshold value according to the second modified example.
Fig. 23 is a diagram illustrating an example of a relation between the determination threshold value and the inertial sensor value according to the second modified example.

### MODE FOR CARRYING OUT THE INVENTION

Hereinafter, an embodiment will be described with reference to the drawings. However, the embodiment described below is merely an example and is not intended to exclude the application of techniques and various modifications not described below. Further, various illustrative embodiments described below may be appropriately combined. In the drawings used in the following embodiments, the same reference numerals denote the same or similar components unless otherwise specified.

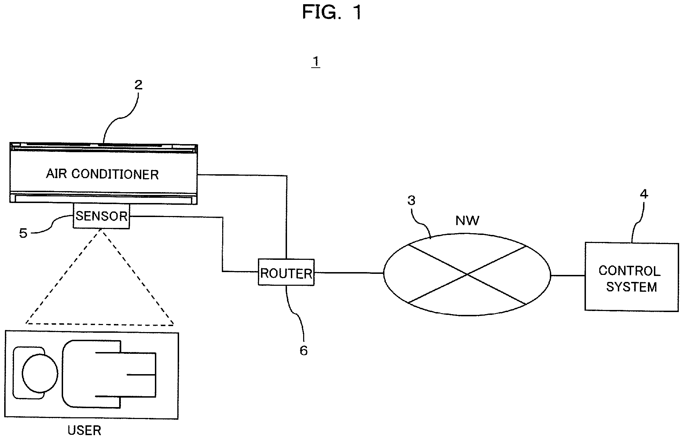

Fig. 1 is a block diagram illustrating a configuration example an air conditioning system according to one embodiment. An air conditioning system 1 illustrated in Fig. 1 may illustratively include an air conditioner 2, a network (NW) 3, and a control system 4.

The air conditioner 2 may be illustratively connected to the network 3 via a router 6 to communicate with each other. The control system 4 may be connected to the network 3. Thus, the air conditioner 2 may illustratively communicate with the control system 4 via the router 6 and the network 3.

The air conditioner 2 can transmit a signal (which may be paraphrased as "information" or "data") representing the operation state of the air conditioner 2 to the control system 4 or receive a signal controlling the operation of the air conditioner 2 from the control system 4 by the communication with the control system 4.

A connection between the air conditioner 2 and the router 6 may be a wired connection or a wireless connection. In other words, the air conditioner 2 may include a communication interface (IF) which supports a communication by one or both of wired and wireless communications.

In addition, the air conditioner 2 may be used for home or business. The home air conditioner 2 is an example of a so-called "home appliance" and "home appliances" which can communicate with the network 3 may be referred to as "information appliances".

The network 3 may illustratively correspond to a WAN (Wide Area Network), a LAN (Local Area Network), or an internet. Further, the network 3 may include a wireless access network. For example, the router 6 can be connected to the wireless access network by a wireless IF to communicate with the control system 4.

As described above, the control system 4 can communicate with the air conditioner 2 via the network 3 and the router 6 and control the operation (also may be referred to as "running") of the air conditioner 2 based on, for example, information received from the air conditioner 2.

The control system 4 may illustratively include one or a plurality of servers. In other words, the operation control of the air conditioner 2 may be controlled by one server or may be controlled in a distributing way by a plurality of servers. For example, the server may correspond to a cloud server provided in a cloud data center.

A sensor 5 may be attached to the air conditioner 2. The sensor 5 can illustratively sense biological information of a user in a non-contact manner in a space corresponding to an air conditioning target of the air conditioner 5 (sic; correctly "air conditioner 2"). In addition, the space corresponding to an air conditioning target of the air conditioner 2 may be referred to as an "air conditioning space" for convenience of description.

The "air conditioning space" may be an indoor space. For example, the indoor space may be a bedroom. The "user" in the "air conditioning space" is an example of the sensing target using the sensor 5. The "biological information" may be referred to as "vital information". The "sensing" may be paraphrased as "detection" or "measurement".

One non-limiting example of the vital information is information representing heartbeat, respiration, or motion of the user. The "motion of the body" of the user may be abbreviated as "body motion" for convenience of description. The "body motion" is not limited to the motion when the user moves and may illustratively include the motion of the body in response to a change in heartbeat or respiration at rest when the user sleeps.

Based on the vital information, for example, a sleep state representing whether the user is sleeping or awakened can be detected, judged, or estimated. Thus, the sensor 5 may be referred to as a "non-contact sleep sensor 5" for convenience of description. A determination on the sleep state based on the vital information may be abbreviated as a "sleep determination" for convenience of description. An example of a sleep determination method will be described below.

The sensor 5 may be connected to the router 6 or may communicate with the network 3 via the router 6 similarly to the air conditioner 2. For example, the sensor 5 may transmit information such as sensed vital information or a sleep determination result to the control system 4 via the router 6 and the network 3.

Information which is transmitted from the sensor 5 to the control system 4 may be generally referred to as "sensor information" for convenience of description. The "sensor information" may include one or both of the vital information and the sleep determination result. The control system 4 may remotely control the operation of the air conditioner 2 so that, for example, a comfortable environment is given to the user in the air conditioning space based on the sensor information.

The remote control of the operation of the air conditioner 2 (which may be referred to as an "air conditioning control") may illustratively include a temperature control, a blowing rate control, and a blowing direction control which help the user to sleep well at bedtime. Such an air conditioning control may be referred to as a "sleep control" for convenience of description.

A connection between the sensor 5 and the router 6 may be a wireless connection or a wired connection. In other words, the sensor 5 may include a communication IF which supports either or both of the wireless communication and the wired communication. In the wireless connection, "WiFi (Wireless Fidelity)" (registered trademark) or "Bluetooth" (registered trademark) may be illustratively used.

Additionally, the sensor 5 does not need to be controlled by the control system 4 differently from the air conditioner 2. In other words, the sensor 5 does not need to support the receiving of the signal transmitted from the control system 4 as long as one-way communication to the control system 4 is possible. In other words, the sensor 5 does not need to communicate with the air conditioner 2 and thus does not need to control the operation of the air conditioner 2.

### (Configuration example of non-contact sleep sensor 5)

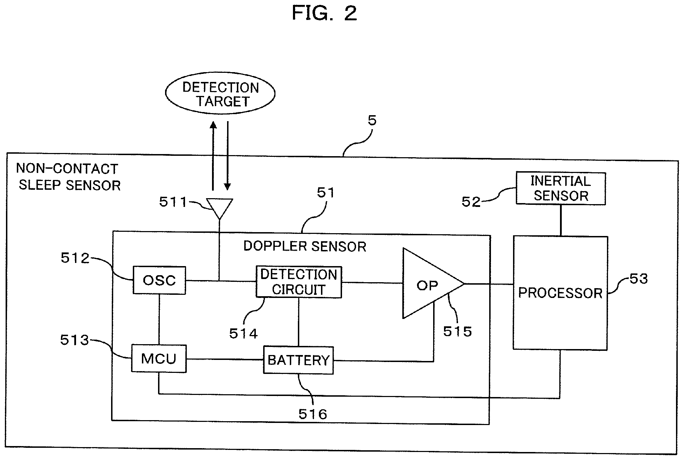

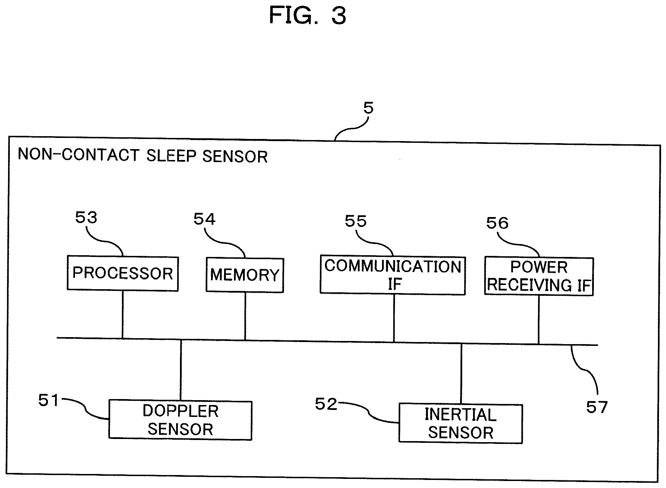

Next, a configuration example of the non-contact sleep sensor 5 will be described with reference to Figs. 2 and 3. As illustrated in Figs. 2 and 3, the non-contact sleep sensor 5 may illustratively include a Doppler sensor 51, an inertial sensor 52, a processor 53, a memory 54, a communication IF 55, and a power receiving IF 56.

As illustrated in Fig. 3, the Doppler sensor 51, the inertial sensor 52, the processor 53, the memory 54, the communication IF 55, and the power receiving IF 56 may be illustratively connected to one another by a bus 57 to communicate with one another. In addition, the non-contact sleep sensor 5 including the Doppler sensor 51 and the inertial sensor 52 may be referred to as the "sensor unit 5".

The Doppler sensor 51 illustratively generates a beat signal by detecting the phases of the radio wave transmitted to the air conditioning space and the reflection wave of the transmission radio wave. The beat signal may be given to the processor 53 in the form of the output signal of the Doppler sensor 51.

For example, as illustrated in Fig. 2, the Doppler sensor 11 may include an antenna 511, a local oscillator (OSC) 512, a MCU (Micro Control Unit) 513, a detection circuit 514, an operational amplifier (OP) 515, and a battery 516.

The antenna 511 transmits a radio wave having an oscillation frequency generated by the OSC 512 to the air conditioning space and receives a radio wave (a reflection wave) in which the transmission radio wave is reflected by the user positioned at the air conditioning space. In the example of Fig. 2, the antenna 511 is commonly used for the transmitting and receiving, but may be separately used for the transmitting and receiving.

The OSC 512 is illustratively oscillated in response to the control of the MCU 513 and outputs a signal of a predetermined frequency (which may be referred to as a "local signal" for convenience of description). The local signal is transmitted as a transmission radio wave from the antenna 511 and is input to the detection circuit 514.

The oscillation frequency (in other words, the frequency of the radio wave transmitted from the Doppler sensor 51) of the OSC 512 may be illustratively a frequency in a microwave band. The microwave band may be illustratively a band of 2.4 GHz or 24 GHz. These frequency bands are examples of the frequency band which is allowed to be used indoors under the Radio Law of Japan. The frequency band which is not regulated by the Radio Law may be used as a transmission radio wave of the Doppler sensor 51.

The MCU 513 illustratively controls the oscillation operation of the OSC 512 in response to the control of the processor 53.

The detection circuit 514 detects the phases of the reflection wave received by the antenna 511 and the local signal (in other words, the transmission radio wave) output from the OSC 512 and outputs a beat signal. In addition, the detection circuit 514 may be replaced by a mixer which mixes the transmission radio wave and the reflection wave. The mixing using the mixer may be understood to be equivalent to the phase detection.

Here, the beat signal which is obtained by the detection circuit 514 has a change in amplitude and frequency caused by a Doppler effect in response to a physical variation such as heartbeat, respiration, or body motion of the user in the air conditioning space.

For example, there is a tendency that the frequency and the amplitude value of the beat signal increase as the physical variation of the user in the air conditioning space (in other words, a relative speed with respect to the Doppler sensor 11) increases. In other words, the beat signal includes information representing a physical variation such as heartbeat, respiration, or body motion of the user.

The operational amplifier 515 amplifies the beat signal output from the detection circuit 514. The amplified beat signal is input to the processor 53.

The battery 516 illustratively supplies driving power to the MCU 513, the detection circuit 514, and the operational amplifier 515.

Meanwhile, the inertial sensor 52 illustratively senses the "motion" (which may be paraphrased as a "positional change") of the non-contact sleep sensor 5 itself. The "motion" of the non-contact sleep sensor 5 may be generated, for example, when the vibration generated during the operation of the air conditioner 2 is transmitted to the non-contact sleep sensor 5.

The vibration during the operation of the air conditioner 2 may be illustratively caused by the rotation of the blowing fan of the air conditioner 2, the motion of the louver of the air conditioner 2, and the air blown out from the louver. The inertial sensor 52 can sense the vibration of the air conditioner 2 caused by these factors as the "motion" of the non-contact sensor 5(sic; correctly "non-contact sleep sensor 5").

When a "motion" occurs in the non-contact sleep sensor 5, a signal component in response to the "motion" may be applied as a noise component to the output signal of the embedded Doppler sensor 51. For example, when the vibration of the air conditioner 2 is transmitted to the sensor 5 so that the non-contact sleep sensor 5 is vibrated, the Doppler sensor 51 is also vibrated.

When the Doppler sensor 51 is vibrated, a change in frequency and amplitude caused by the vibration of the Doppler sensor 51 itself is included as noise in the output signal as well as a change in frequency and amplitude in response to the "motion" of the sensing target. For example, there is a tendency that the output amplitude of the Doppler sensor 51 increases in response to the vibration of the Doppler sensor 51 so that the frequency increases.

In addition, since the air conditioner 2 may automatically control the blowing rate or the blowing direction in response to the temperature or the humidity of the air conditioning space during an operation, there is no guarantee that the vibration of the air conditioner 2 during an operation is constant and that the vibration transmitted to the sensor 5 is also constant. For that reason, there is no guarantee that the output amplitude and the frequency of the Doppler sensor 51 are constant during the operation of the air conditioner 2.

When a noise component caused by the vibration of the air conditioner 2 is applied to the output signal of the Doppler sensor 51, the detection accuracy of the vital information of the sensing target may be degraded. As a result, the sleep determination accuracy based on the vital information may be also degraded.

Here, in the embodiment, degradation in detection accuracy of the vital information caused by the noise component in response to the motion of the sensor 5 itself and furthermore degradation in sleep determination accuracy is avoided or inhibited by using the sensing result of the inertial sensor 52. The detail will be described later.

The inertial sensor 52 may be an acceleration sensor or a gyroscope. Any sensor of a piezoelectric type or a capacitance type may be illustratively applied to the acceleration sensor. Any sensor of a rotary machine (rotor) type, an optical type, or a vibration type may be applied to the gyroscope.

The inertial sensor 52 may include one or a plurality of detection axes. The "motion" in a direction aligned to the detection axis may be detected as, for example, an "acceleration". At least one detection axis of the inertial sensor 52 may be aligned to the direction of the directivity of the transmission radio wave of the Doppler sensor 51 (which may be referred to as a "radio wave transmission direction" for convenience of description).

In other words, the inertial sensor 52 may be disposed and set to detect the "motion" of the non-contact sleep sensor 5 with respect to the radio wave transmission direction of the Doppler sensor 51. A signal in response to the "motion" detected by the inertial sensor 52 may be input to the processor 53. In addition, the inertial sensor 52 may be operated during the operation of the Doppler sensor 51.

The processor 53 can detect the vital information of the user in the air conditioning space based on the output signal of the Doppler sensor 51 and the output signal of the inertial sensor 52 and can determine whether the user is sleeping based on the vital information.

In addition, the processor 53 is an example of a calculator having a calculation ability. The calculator may be referred to as a calculation device or a calculation circuit. A CPU (Central Processing Unit) or a DSP (Digital Signal Processor) may be illustratively applied to the processor 53 which is an example of the calculator.

The output signal of the inertial sensor 52 may be used to correct the vital information or the threshold value used in the sleep determination of the processor 53. A detailed example of the correction will be described below.

Next, in Fig. 3, the memory 54 is an example of a storage medium and may be a RAM (Random Access Memory) or a flash memory. The memory 54 may store a program or data which is read by the processor 53 to be operated. The "program" may be referred to as "software" or "application". The "data" may include data which is generated in response to the operation of the processor 53.

The communication IF 55 is illustratively connected to the router 6 so as to communicate with the control system 4 via the network 3. For example, the communication IF 55 may transmit the sensor information of the non-contact sleep sensor 5 (which is illustratively vital information or a sleep determination result) obtained based on the output signal of the Doppler sensor 51 and the output signal of the inertial sensor 52 to the control system 4. Thus, the communication IF 55 is an example of a transmitter which transmits information to the control system 4 by focusing on a transmitting process.

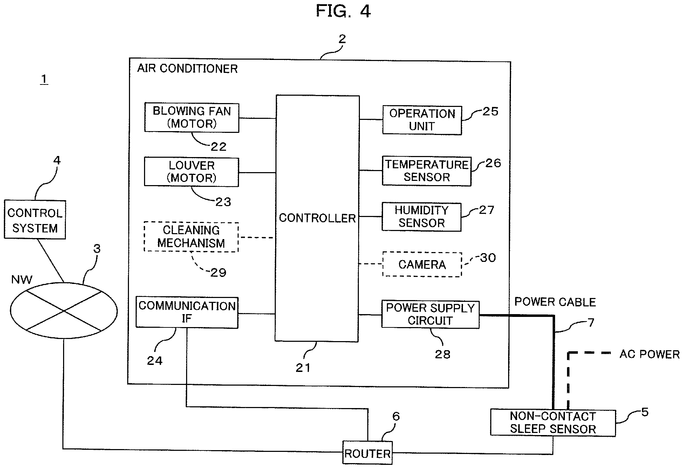

The power receiving IF 56 is illustratively an interface which receives a driving power for driving the non-contact sleep sensor 5. The power receiving IF 56 may be connected to the power supply circuit 28 of the air conditioner 2 by the power cable 7 as indicated by a thick solid line of Fig. 4 so as to receive power from the air conditioner 2. Alternatively, the received power IF 56 may be connected to an AC power supply so as to receive power therefrom as indicated by a thick dotted line of Fig. 4.

In other words, a power supply for the non-contact sleep sensor 5 may be shared by the air conditioner 2 or may be separated from the air conditioner 2. When power is supplied from a power supply separated from the air conditioner 2 to the non-contact sleep sensor 5, the non-contact sleep sensor 5 can perform a sensing operation even when the power of the air conditioner 2 is turned off. In other words, since the non-contact sleep sensor 5 can be operated as a single sensor 5 even when the air conditioner 2 is not operated, the sensor can be used as a "watching function".

In addition, an universal serial bus (USB) may be used for the connection between the received power IF 56 of the non-contact sleep sensor 5 and the power supply circuit 28 of the air conditioner 2. For example, the air conditioner 2 may include an USB port which can supply power. The received power IF 56 of the non-contact sleep sensor 5 may receive power while being connected to the USB port of the air conditioner 2 by a USB cable which is an example of the power cable 7.

### (Configuration example of air conditioner 2)

Fig. 4 is a block diagram illustrating a configuration example of the air conditioning system 1 by focusing on the configuration of the air conditioner 2. The air conditioner 2 illustrated in Fig. 4 illustratively includes a controller 21. The controller 21 controls the operation of the air conditioner 2.

A motor for driving a blowing fan 22 of the air conditioner 2 or a motor for driving a louver 23 of the air conditioner 2 may be illustratively connected to the controller 21. The blowing fan 22 is an example of a blowing machine and may be illustratively a cross flow fan. The louver 23 is an example of a wind direction adjuster and may be referred to as an "air wing 23".

When the cross flow fan 22 is controlled by the controller 21, for example, the blowing rate of the air conditioner 2 can be controlled. When the air wing 23 is controlled by the controller 21, for example, the blowing direction of the air conditioner 2 can be controlled.

Further, a communication IF 24, an operation unit 25, a temperature sensor 26, a humidity sensor 27, and the power supply circuit 28 may be illustratively connected to the controller 21.

The communication IF 24 is an interface which is connected to the router 6 and can communicate with the control system 4 via the network 3. An Ethernet (registered trademark) card may be illustratively applied to the communication IF 24.

The communication IF 24 is an example of a transmitter which transmits information to the control system 4 by focusing on a transmitting process and is an example of a receiver which receives information transmitted from the control system 4 to the air conditioner 2 by focusing on a receiving process.

The operation unit 25 is operated by the user of the air conditioner 2 and a signal in response to the operation (which may be referred to as an "operation signal" for convenience of description) is input to the controller 21. A control in response to the operation signal is performed by the controller 21.

In addition, the operation unit 25 may correspond to an operation panel attached to the body of the air conditioner 2 or may correspond to a remote controller for remotely operating the air conditioner 2 by, for example, an infrared communication.

The temperature sensor 26 senses the temperature of the air conditioning space. The humidity sensor 27 senses the humidity of the air conditioning space. The controller 21 may adaptively control the blowing fan 22 or the louver 23 based on the sensor information of either or both of the temperature sensor 26 and the humidity sensor 27.

The power supply circuit 28 generates driving power for driving the air conditioner 2. As described above, power may be supplied from the power supply circuit 28 to the non-contact sleep sensor 5 through the power cable 7.

In addition, a cleaning mechanism 29 may be connected to the controller 21. The cleaning mechanism 29 may be illustratively a mechanism for autonomously cleaning the filter of the air conditioner 2 by the air conditioner 2. A cleaning operation using the cleaning mechanism 29 may be illustratively performed in response to the power-off state of the air conditioner 2.

Further, a camera 30 may be connected to the controller 21. The camera 30 may capture the image of the air conditioning space. The image data which is captured by the camera 30 may be included in information transmitted from the communication IF 24 to the control system 4. The image data may be still image data or moving image data.

The image data of the camera 30 which is received by the control system 4 may be accessed from an information terminal. The information terminal may be, for example, a terminal possessed by a user of the air conditioner 2 or its relatives, or a terminal possessed by a security company permitted to monitor the air conditioning space. A personal computer (PC), a cellular phone (which may include a smart phone), a tablet PC, or the like may correspond to the information terminal.

By referring to the image data of the air conditioning space received by the control system 4 through the information terminal, it is possible to monitor and confirm the state of the air conditioning space at a remote place away from the air conditioning space by the user of the air conditioner 2, its relatives, security companies, or the like.

### (Configuration example of control system 4)

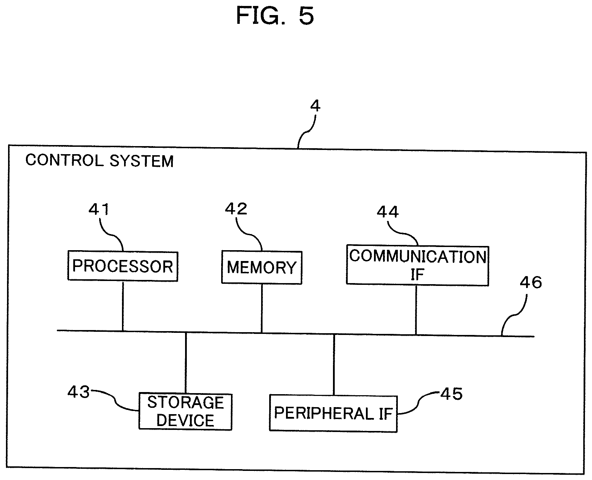

Fig. 5 is a block diagram illustrating a configuration example of the control system 4 illustrated in Fig. 1. The control system 4 illustrated in Fig. 5 may illustratively include a processor 41, a memory 42, a storage device 43, a communication interface (IF) 44, and a peripheral IF 45.

The processor 41, the memory 42, the storage device 43, the communication IF 44, and the peripheral IF 45 may be illustratively connected to one another via a bus 46 to communicate with one another.

The processor 41 illustratively controls the operation of the control system 4. The control may include a control of a communication with the network 3 or a remote control of the air conditioner 2 via the network 3 as described above.

For example, the processor 41 may generate a control signal for controlling the operation of the air conditioner 2 based on the sensor information of the non-contact sleep sensor 5 received by the communication IF 44. The control signal may be transmitted from the communication IF 44 to the air conditioner 2. The control signal which is transmitted to the air conditioner 2 may be received by the air conditioner 2 (for example, the communication IF 24) via the network 3 and the router 6.

In addition, the processor 41 is an example of a calculator having a calculation ability similarly to the processor 53 of the non-contact sleep sensor 5. The calculator may be referred to as a calculation device or a calculation circuit. A CPU or DSP may be illustratively applied to the processor 41 which is an example of the calculator.

The memory 42 is an example of a storage medium and may be a RAM or a flash memory. A program or data which is used for a reading operation by the processor 41 may be stored in the memory 42. The "program" may include a program for remotely controlling the operation of the air conditioner 2. The "data" may include data generated in response to the operation of the processor 42(sic; correctly "processor 41") or the control signal to the air conditioner 2.

The storage device 43 may illustratively store the sensor information of the non-contact sleep sensor 5 received by the communication IF 44. The sensor information may be illustratively collected into a database (DB) in the storage device 43. The DB data may be referred to as "cloud data" or "big data". In addition, a hard disk drive (HDD) or a solid state drive (SSD) may be illustratively applied to the storage device 43.

The communication IF 44 is illustratively connected to the network 3 so as to communicate with the air conditioner 2 via the network 3. The communication IF 44 is an example of a receiver which receives information transmitted from the non-contact sleep sensor 5 to the control system 4 by focusing on a receiving process. Meanwhile, the communication IF 44 is, for example, an example of a transmitter which transmits the control signal to the air conditioner 2 generated by the processor 41 by focusing on a transmitting process. An Ethernet (registered trademark) card may be illustratively applied to the communication IF 44.

The peripheral IF 45 is illustratively an interface which connects the peripheral device to the control system 4. The peripheral device may include an input device which inputs information to the control system 4 or an output device which outputs information obtained by the control system 4. The input device may include a keyboard, a mouse, a touch panel, or the like. The output device may include a display, a printer, or the like.

### (Attachment position of non-contact sleep sensor 5)

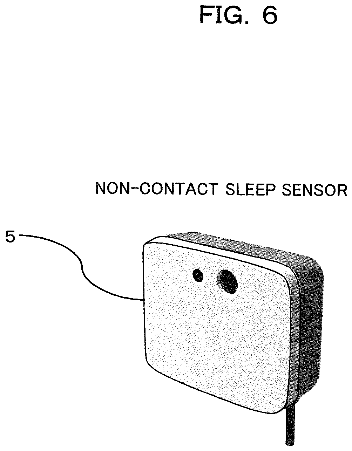

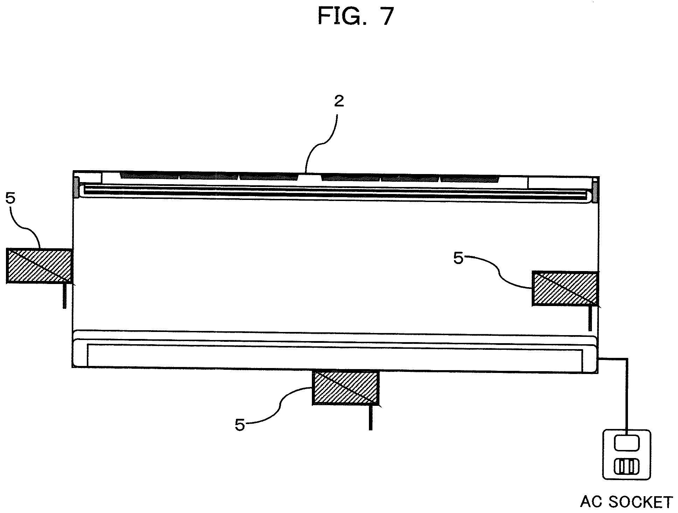

Next, an example of an attachment position of the non-contact sleep sensor 5 will be described with reference to Figs. 6 to 9. Fig. 6 is a diagram schematically illustrating an example of an appearance of the non-contact sleep sensor 5. As schematically illustrated in Fig. 7, the non-contact sleep sensor 5 illustrated in Fig. 6 may be attached to any position of a surface of a casing of the body of the air conditioner 2.

The casing surface is an example of a position which is vibrated during the operation of the air conditioner 2. In other words, the non-contact sleep sensor 5 may be attached to a position which receives a vibration during the operation of the air conditioner 2. The vibration during the operation of the air conditioner 2 may be illustratively generated in response to the operation of the above-described blowing fan 22, the louver 23, or the cleaning mechanism 29.

The casing surface to which the non-contact sleep sensor 5 is attached may be any one of a front surface of the casing of the body of the air conditioner 2, a side surface of the casing, and a bottom surface of the casing, for example, as schematically illustrated in Fig. 7. As long as the attachment position is a position in which the air conditioning space can be seen by the non-contact sleep sensor 5, the attachment position of the non-contact sleep sensor 5 with respect to the casing surface of the body of the air conditioner 2 may be arbitrarily set.

For example, the body of the air conditioner 2 may be installed at a position close to an indoor wall surface or may be installed at a position separated from the indoor wall surface. Further, the body of the air conditioner 2 may be installed at a position in the vicinity of the center between both facing indoor wall surfaces.

Thus, the attachment position of the non-contact sleep sensor 5 with respect to the casing surface of the body of the air conditioner 2 may be appropriately selected in response to the installation position (which may be referred to as an "installation environment" or an "installation condition") of the body of the air conditioner 2.

As one non-limiting example, since the vicinity of the center in the width direction of the bottom surface of the casing of the body of the air conditioner 2 is a position in which the air conditioning space can be seen in many cases when the air conditioner 2 is of a "wall mount type", the non-contact sleep sensor 5 may be attached to such a position.

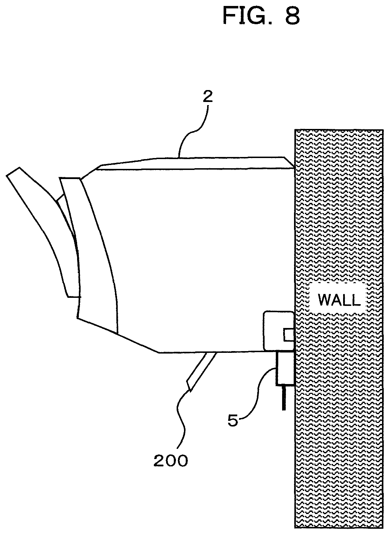

In addition, Fig. 8 is a side view schematically illustrating an example in which the non-contact sleep sensor 5 is attached to the bottom surface of the casing of the body of the air conditioner 2. As illustrated in Fig. 8, when an open/close door 200 of an outlet is provided in the bottom surface of the casing of the air conditioner 2, the non-contact sleep sensor 5 may be attached to a position avoiding the open/close door 200. The position avoiding the open/close door 200 may be a position close to the wall surface provided with the air conditioner 2 in the bottom surface of the casing of the body of the air conditioner 2 in the example of Fig. 8.

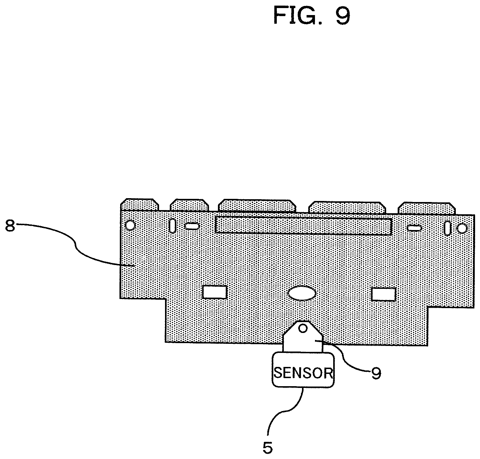

The non-contact sleep sensor 5 may be attached to a jig 8 (which may be referred to as an "attachment jig 8") for attaching the body of the air conditioner 2 to the wall surface, for example, as schematically illustrated in Fig. 9 while the attachment position is not limited to the casing surface of the body of the air conditioner 2.

Since a vibration during the operation of the air conditioner 2 is transmitted to the jig 8, the non-contact sleep sensor 5 which is attached to the jig 8 receives the vibration during the operation of the air conditioner 2. In other words, the non-contact sleep sensor 5 may be attached to a position contacting the jig 8 to which the body of the air conditioner 2 is attached.

In addition, the jig 8 may be understood as an element of the air conditioner 2. In other words, it may be understood that a set of the body of the air conditioner 2 and the jig 8 integrally constitute the air conditioner 2. The set of the body of the air conditioner 2 and the jig 8 may be paraphrased as the "air conditioning unit 2" for convenience of description.

The non-contact sleep sensor 5 may be attached in a separable manner. Illustratively, bonding means such as an adhesive, a double-sided tape, and a screw may be applied to the attachment of the non-contact sleep sensor 5.

For example, the non-contact sleep sensor 5 may be attached to the casing surface of the body of the air conditioner 2 by an adhesive, a double-sided tape, or a screw. Further, the non-contact sleep sensor 5 may be attached to the jig 8 by an adhesive or a double-sided tape or may be screwed to a jig 9 attaching the sensor 5 to the jig 8 as schematically illustrated in Fig. 9.

In addition, the non-contact sleep sensor 5 may be attached into the air conditioner 2 instead of the casing surface of the body of the air conditioner 2. For example, the sensor 5 may be attached to a rear surface of a front cover of the air conditioner 2 or a frame or a component existing in a space inside the front cover. In any of the above-described attachment methods, the attachment of the sensor 5 may be "retrofitting".

The air conditioner 2 is not limited to the air conditioner of the "wall mount type" and may be the air conditioner of the type in which the air conditioner is attached to the "ceiling" of the air conditioning space (which may be referred to as a "ceiling built type" for convenience of description). Even in the air conditioner 2 of the "ceiling built type", the non-contact sleep sensor 5 may be attached to a position which receives the vibration during the operation of the air conditioner 2.

### (Operation example)

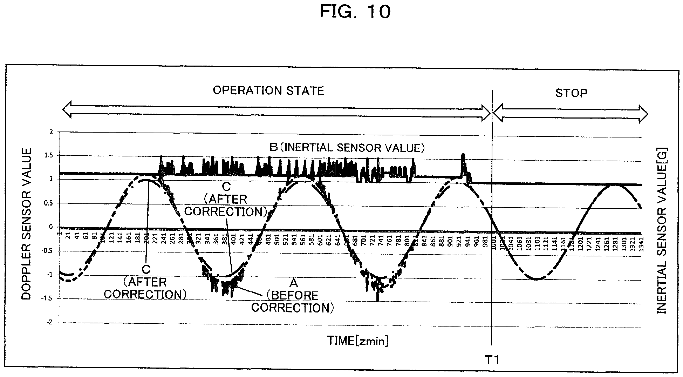

Hereinafter, an operation example of the air conditioning system 1 will be described with reference to Figs. 10 to 18. Fig. 10 is a diagram illustrating an example of a change in time of output signals (which may be referred to as "detected values") of the Doppler sensor 51 and the inertial sensor 52 of the non-contact sleep sensor 5.

A horizontal axis of Fig. 10 indicates a time, a vertical axis at the left side of Fig. 10 indicates the detected value (for example, a normalized voltage value) of the Doppler sensor 51, and a vertical axis at the right side of Fig. 10 indicates the detected value (for example, acceleration [G]) of the inertial sensor 52. The detected value of the Doppler sensor 51 may be referred to as a "Doppler sensor value" for convenience of description. The detected value of the inertial sensor 52 may be referred to as an "inertial sensor value" for convenience of description.

In Fig. 10, a signal waveform indicated by a dotted line A indicates the detected value of the Doppler sensor 51 and a signal waveform indicated by a solid line B indicates the detected value of the inertial sensor 52. In addition, a signal waveform indicated by a one-dotted chain line C indicates a detected value (which may be referred to as a "corrected detected value") obtained by correcting the detected value (the dotted line A) of the Doppler sensor 51 by a correction process to be described later. Further, Fig. 10 illustrates an example in which the air conditioner 2 is operated until a time T1 and the air conditioner 2 is turned off at the time T1 so that the operation of the air conditioner 2 is stopped.

As illustrated in Fig. 10, since a vibration associated with the operation of the air conditioner 2 is transmitted to the non-contact sleep sensor 5 during the operation of the air conditioner 2 until the time T1, a change in response to the vibration occurs in the Doppler sensor value (see the dotted line A) and the inertial sensor value (see the solid line B).

In addition, when the air conditioner 2 is not operated and vibrated, a change in inertial sensor value does not occur. For example, an acceleration which is detected by the inertial sensor 52 when the air conditioner 2 is not operated may be considered as a 1 gravity acceleration (1 G).

As indicated by the dotted line A of Fig. 10, a change in Doppler sensor value in response to the vibration during the operation of the air conditioner 2 becomes a noise component (which may be referred to as "vibration noise" for convenience of description) for the original Doppler sensor value.

As described above, the vibration noise leads to a detection error of the vital information based on the Doppler sensor value. For that reason, an error may occur in the sleep determination based on the vital information. The noise component which may occur in the Doppler sensor value due to the vibration during the operation of the air conditioner 2 can be canceled by using the inertial sensor value.

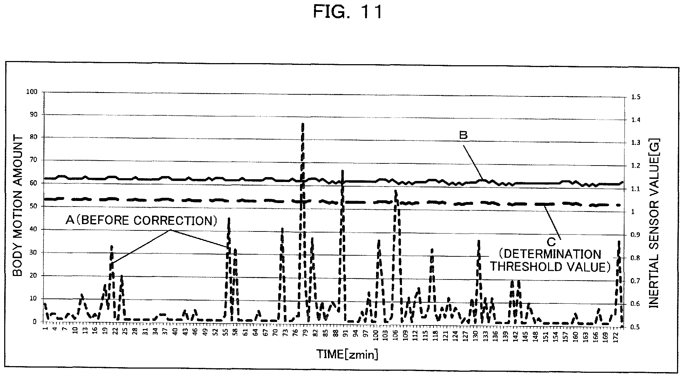

Fig. 11 is a diagram illustrating an example of a change in body motion amount with time and a change in inertial sensor value with time as an example of the vital information obtained based on the Doppler sensor value. A vertical axis at the left side of Fig. 11 indicates the body motion amount and a vertical axis at the right side of Fig. 11 indicates the inertial sensor value. In Fig. 11, a signal waveform indicated by a dotted line A indicates an example of a change in body motion amount with time and a signal waveform indicated by a solid line B indicates an example of a change in inertial sensor value (amplitude value) with time.

In Fig. 11, a dotted line C indicates a threshold value (which may be referred to as a "determination threshold value") used in the sleep determination based on the body motion amount. Illustratively, it may be determined that the user in the air conditioning space is awakened when the body motion amount exceeds the determination threshold value and it may be determined that the user is sleeping when the body motion amount is smaller than the determination threshold value.

The body motion amount can be understood as a change in Doppler sensor value with time. For example, when the user corresponding to the sensing target is awakened and active, the body motion of the sensing target appears as a change in amplitude value and frequency in the Doppler sensor value. For example, there is a tendency that the amplitude value and the frequency of the Doppler sensor value increase as the body motion amount of the user increases.

When the user sleeps at rest, a change in heartbeat or respiration dominantly occurs in the body motion of the user. For that reason, the amplitude value of the Doppler sensor value does not change or may be considered to be a negligible change even if there is a change.

Thus, it may be considered that the body motion caused by a change in heartbeat or respiration appears as a change in frequency of the Doppler sensor value. For example, there is a tendency that the frequency of the Doppler sensor value increases as the heart rate or respiration rate increases.

Thus, the body motion amount can be detected based on a change in amplitude value and frequency of the Doppler sensor value. A change in amplitude value and frequency of the Doppler sensor value can be understood as a change in length, for example, when the signal waveform (see the dotted line A) illustrated in Fig. 10 is linearly extended in a time domain.

A length at the time when the signal waveform is linearly extended in the time domain may be referred to as an "extended wavelength" for convenience of description. Thus, the "extended wavelength" is a concept different from a normal "wavelength". The "extended wavelength" may be considered to be equivalent to a length of a locus which is drawn by the Doppler sensor value in the time domain for a certain unit time. In addition, the unit time may be a unit of a "second" or a "minute".

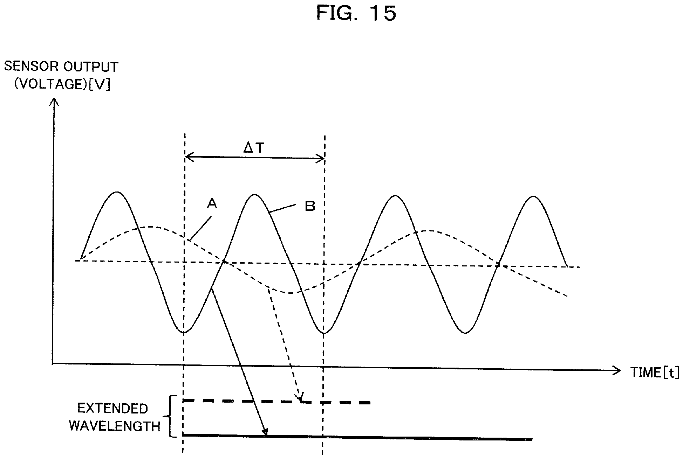

Fig. 15 schematically illustrates a concept of the "extended wavelength". A horizontal axis of Fig. 15 indicates a time (t) and a vertical axis of Fig. 15 indicates the Doppler sensor value (for example, a voltage [V]).

In Fig. 15, a signal waveform indicated by a dotted line A illustratively and schematically indicates a change in Doppler sensor value with time when the user of the sensing target is sleeping. A signal waveform indicated by a solid line B schematically indicates a change in Doppler sensor value with time when the user of the sensing target is awakened and active.

The "extended wavelength" corresponds to a length at the time when a signal waveform per unit time (ΔT) indicated by the dotted line A and the solid line B at the lower side of Fig. 15 is linearly extended in a time domain.

The "extended wavelength" can be illustratively calculated by sequentially storing the Doppler sensor value in the memory 54 (see Fig. 3) with a certain period (which may be referred to as a "sampling period") and adding an amplitude value change amount for the unit time.

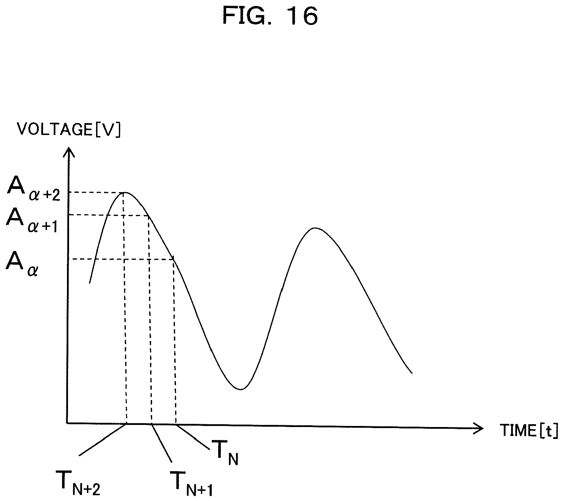

A calculation example of the "extended wavelength" will be described with reference to Fig. 16. A horizontal axis of Fig. 16 indicates a time (t) and a vertical axis of Fig. 16 indicates the Doppler sensor value (for example, a voltage [V] corresponding to the amplitude value).

In the signal waveform illustrated in Fig. 16, the Doppler sensor values at certain timings t = T_{N+2}, t = T_{N+1}, and t = T_{N} are respectively "A_{α+2}", "A_{α+1'}" and "A_{α}".

In addition, "N" is an integer which indicates a timing label. "A" is a real number for the voltage value [V] and "α" is an integer indicating the label of the voltage value. The timings t = T_{N+2}, t = T_{N+1}, and t = T_{N} may be respectively referred to as the "sampling timings". The interval of the sampling timing may be constant or different.

The processor 53 illustratively obtains the amplitude change amount between the sampling timings based on the amplitude value (the voltage value) obtained at each sampling timing. For example, the processor 53 may obtain a difference in amplitude value at the adjacent sampling timings as an amplitude change amount between the sampling timings.

Illustratively, the processor 53 may obtain the amplitude change amount between the sampling timing t = T_{N+2} and the next sampling timing t = T_{N+1} as an absolute value |A_{α+1} - A_{α+2}| . Similarly, the processor 53 may obtain the amplitude change amount between the sampling timing t = T_{N+1} and the next sampling timing t = T_{N} as an absolute value |A_{α} - A_{α+1}|.

The processor 53 can calculate the "extended wavelength" by repeating such a calculation for the number of sampling times per unit time and adding the amplitude change amounts like |A_{α} - A_{α+1}| + |A_{α+1} - A_{α+2}| + .... Also in the inertial sensor value detected by the inertial sensor 52, the "extended wavelength" can be calculated in the same way.

In addition, as illustrated in Fig. 16, when the Doppler sensor value is indicated by the voltage value [V], the unit of the "extended wavelength" is expressed by, for example, "voltage/time" (V/min).

Further, the calculation accuracy of the "extended wavelength" is degraded when the number of samplings of the amplitude value per unit time is too small and a calculation delay occurs due to an increase in calculation load when the number of samplings is large. For this reason, the number of samplings may be set in a realistic range. Further, the "extended wavelength" may be averaged over a predetermined time. For example, an average of sixty "extended wavelengths" obtained for one minute may be obtained while the unit time is one second.

A change in the "extended wavelength" per unit time may be detected as, for example, the "body motion amount" indicated by the dotted line A of Fig. 11. For example, a value obtained by adding the "extended wavelength" obtained every second for one minute (= sixty seconds) may be obtained as the "body motion amount".

The sleep determination may be performed based on the "body motion amount" obtained in this way. An arithmetic expression (may be referred to as a "determination expression") called "AW2 expression" or "Cole expression" may be illustratively applied to the sleep determination.

For example, "sleeping" may be determined when a calculation value obtained by "AW2 expression" or "Cole expression" based on the "body motion amount" for a certain time (illustratively, several minutes) is equal to or larger than a certain threshold value and "awakening" may be determined when the calculation value is smaller than the threshold value. In addition, the calculation value obtained by "AW2 expression" or "Cole expression" may be referred to as a "determination value".

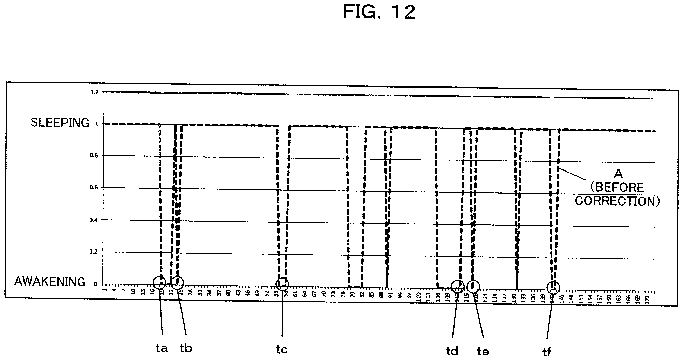

Figs. 12 to 14 illustrate an example of a change in time of the calculation value (the determination value) obtained based on the body motion amount. Figs. 12 to 14 illustrate an example in which "awakening" is determined in the case of the "determination value = 0" and "sleeping" is determined in the case of the "determination value = 1". A numerical value of a horizontal axis (a time) of each of Figs. 12 to 14 corresponds to a numerical value of a horizontal axis of Fig. 11.

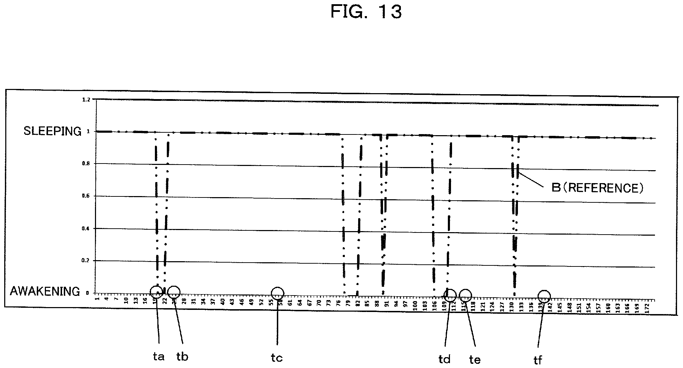

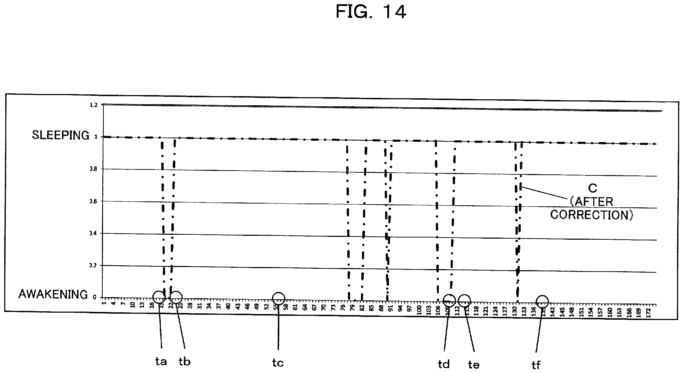

A dotted line A illustrated in Fig. 12 indicates a determination value before the correction by the inertial sensor value, a two-dotted chain line B illustrated in Fig. 13 indicates a reference of the determination value, and a one-dotted chain line C illustrated in Fig. 14 indicates a determination value after the correction by the inertial sensor value. The reference (the two-dotted chain line B) of the determination value illustrated in Fig. 13 illustratively corresponds to a determination value on the assumption that the air conditioner 2 is not operated and the inertial sensor value does not change.

From the comparison of Figs. 12 and 13, it is understood that a deviation from the reference (the two-dotted chain line B) of Fig. 13 occurs in the determination value (the dotted line A) of Fig. 12 at six positions (the determination timings ta to tf) of the time domain in Fig. 12. For example, an erroneous determination of "awakening" is made in Fig. 12 at the determination timings ta to tf instead of "sleeping" according to the reference of Fig. 13.

Since the noise component in response to the vibration during the operation of the air conditioner 2 is added to the Doppler sensor value from the comparison of Figs. 10 to 13, it can be understood that the erroneous determination is made due to an error with respect to the reference in the determination value.

Thus, when the vibration noise is canceled from the Doppler sensor value (or may be the "extended wavelength") by using the inertial sensor value, the determination value can match or approach the reference of Fig. 13 as indicated by the one-dotted chain line C of Fig. 14. Thus, the sleep determination accuracy can be improved.

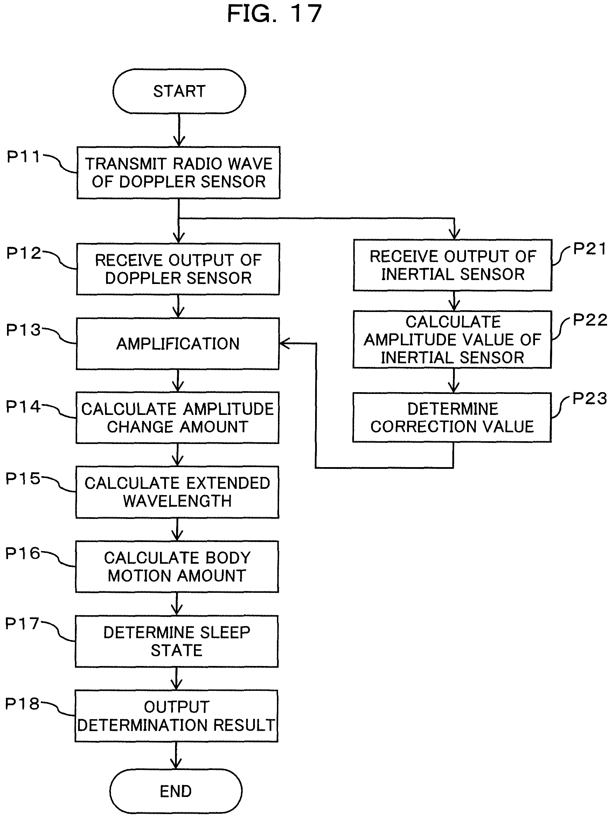

Hereinafter, an example of a sleep determination process including a process of canceling the vibration noise of the Doppler sensor value will be described with reference to Fig. 17. In addition, a flowchart illustrated in Fig. 17 may be illustratively understood while being performed in the processor 53 of the non-contact sleep sensor 5.

The processor 53 transmits a radio wave from the Doppler sensor 51 to the air conditioning space during the operation of the air conditioner 2 (process P11). In addition, the Doppler sensor 51 may be controlled to transmit the radio wave to the air conditioning space during at least the operation of the air conditioner 2. For example, the Doppler sensor 51 may be controlled to transmit a radio wave at all times regardless of whether the air conditioner 2 is operated or stopped.

In response to the transmission of the radio wave by the Doppler sensor 51, the processor 53 receives the Doppler sensor value from the Doppler sensor 51 (process P12). Further, the processor 53 receives the inertial sensor value from the inertial sensor 52 (process P21).

The processor 53 may appropriately amplify the received Doppler sensor value. The amplification factor of the amplification may be corrected in response to the inertial sensor value (process P13). In other words, the Doppler sensor value may be corrected in response to the inertial sensor value.

For example, the processor 53 calculates the amplitude value of the inertial sensor value (process P22). The amplitude value of the inertial sensor value may be calculated from the detected value of one detection axis aligned to the radio wave transmission direction of the Doppler sensor 51 or may be calculated as a composite value of the detected values of the detection axes including the corresponding detection axis.

The processor 53 determines the correction value in response to the calculated amplitude value (process P23). The correction value corresponds to a value which can cancel (or minimize) the vibration noise applied to the Doppler sensor value. For example, the processor 53 may determine the correction value so that the Doppler sensor value relatively decreases as the inertial sensor value increases.

Thus, in the process P15, the processor 53 can calculate the "extended wavelength" in which the vibration noise is canceled. The processor 53 calculates the amplitude change amount based on the corrected Doppler sensor value as described in Fig. 15 (process P14).

In addition, the correction in response to the inertial sensor value may be applied to the amplitude change amount. Further, the process P12 and the processes P21 to P23 may be performed together.

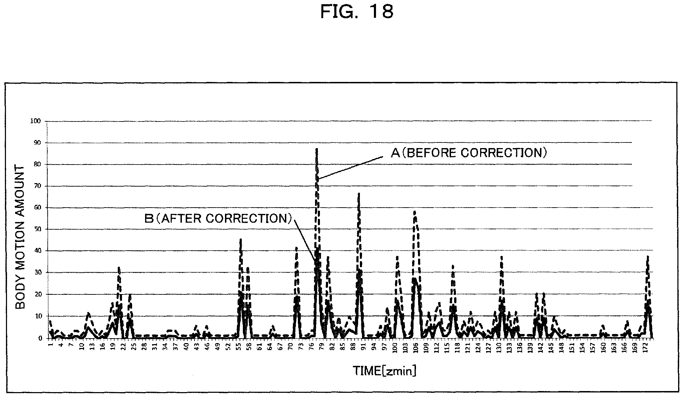

The processor 53 calculates the "extended wavelength" for the Doppler sensor value based on the amplitude change amount calculated in the process P14 (process P15) and calculates the "body motion amount" as described above based on the calculated "extended wavelength" (process P16). Fig. 18 illustrates an example of a change in time of the body motion amount before and after the correction using the inertial sensor value.

In Fig. 18, a dotted line A illustrates an example of a change in time of the body motion amount before the correction and the solid line B illustrates an example of a change in time of the body motion amount after the correction. As illustrated in Fig. 18, the amplitude value of the body motion amount after the correction becomes smaller than the amplitude value of the body motion amount before the correction in response to the cancelation of the vibration noise.

The processor 53 performs the sleep determination illustrated in Figs. 12 to 14 based on the "calculated body motion amount" (process P17). The sleep determination result may be illustratively transmitted from the communication IF 55 to the control system 4 (process P18).

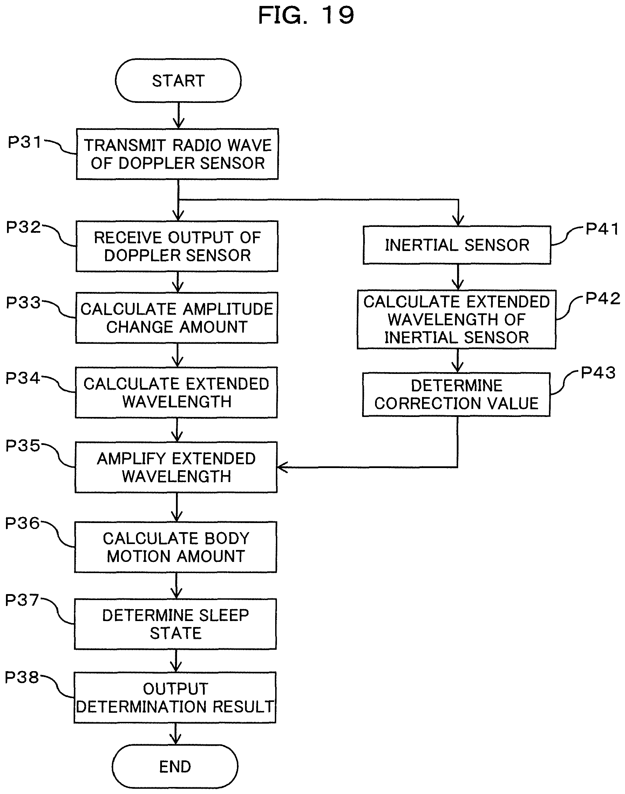

As described above, since the Doppler sensor value is corrected in response to the inertial sensor value in the process P13, a subsequent process does not need to be modified compared to first and second modified examples to be described later (Figs. 19 to 23).

### (First Modified Example)

In addition, the vibration noise may be canceled by correcting the "extended wavelength" of the Doppler sensor value, for example, as illustrated in Fig. 19 instead of correcting the Doppler sensor value (or the amplitude change amount). In addition, the processes P31 to P33, P36 to P38, and P41 of Fig. 19 may be respectively the same as the processes P11, P12, P14, P16 to P18, and P21 of Fig. 17.

As illustrated in Fig. 19, the processor 53 transmits a radio wave from the Doppler sensor 51 to the air conditioning space during, for example, the operation of the air conditioner 2 (process P31).

In response to the transmission of the radio wave using the Doppler sensor 51, the processor 53 receives the Doppler sensor value from the Doppler sensor 51 (process P32). Further, the processor 53 receives the inertial sensor value from the inertial sensor 52 (process P41).

The processor 53 calculates the amplitude change amount based on the Doppler sensor value received from the Doppler sensor 51 (process P33) and calculates a "first extended wavelength" based on the calculated amplitude change amount (process P34).

The "calculated extended wavelength" may be appropriately amplified (process P35) and the amplification factor of the amplification may be corrected based on the inertial sensor value.

For example, the processor 53 calculates a "second extended wavelength" based on the inertial sensor value received from the inertial sensor 52 (process P42) and determines a correction value in response to the "extended wavelength" (process P43) .

The correction value corresponds to a value which can cancel (or minimize) the vibration noise applied to the "extended wavelength" of the Doppler sensor value. For example, the processor 53 may linearly determine the correction value so that the extended wavelength of the Doppler sensor value appears to be relatively smaller as the extended wavelength of the inertial sensor value becomes longer.

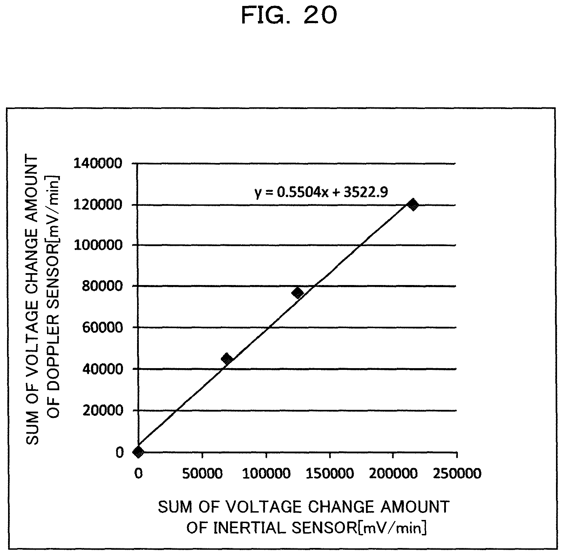

As one non-limiting example, the processor 53 may obtain a correction value Y in a linear calculation expressed by y = ax + b (a and b are constants) when the "extended wavelength" of the inertial sensor value is indicated by "x" and the correction value of the extended wavelength of the Doppler sensor value is indicated by "y". Fig. 20 illustrates an example of a relation of y = ax + b.

A horizontal axis of Fig. 20 indicates the sum of the amplitude change amount (the voltage change amount) for the unit time of the inertial sensor value and corresponds to the extended wavelength of the inertial sensor value. A vertical axis of Fig. 20 indicates the sum of the amplitude change amount (the voltage change amount) for the unit time of the Doppler sensor value and indicates the extended wavelength (the correction value) of the Doppler sensor value. In the example of Fig. 20, a constant a is 0.5504 and a constant b is 3522.9.

In the process P35, the processor 53 can cancel the vibration noise from the "extended wavelength" by correcting the amplification factor of the "extended wavelength" of the Doppler sensor value according to the correction value. The correction may be considered to be equivalent to the subtraction of the above-described correction value y from the extended wavelength of the Doppler sensor value. In addition, the processes P32 to P34 and the processes P41 to P43 may be performed together.

The processor 53 calculates the "body motion amount" based on the "extended wavelength" of which the vibration noise is canceled in the process P35 (process P36) and performs the sleep determination illustrated in Figs. 12 to 14 based on the "calculated body motion amount" (process P37).

According to the first modified example, since the "extended wavelength" of the Doppler sensor value is corrected in response to the "extended wavelength" of the inertial sensor value, a highly accurate correction can be performed compared to a case where the Doppler sensor value is corrected in response to the inertial sensor value as described above.

For example, when an average value for a unit time of sixty seconds is used in the calculation of the "extended wavelength" of the Doppler sensor value or the calculation of the "body motion amount" based on the "extended wavelength" in the case where the Doppler sensor value is corrected in response to the inertial sensor value, an error may be accumulated. This point is also the same in the second modified example to be described later.

In contrast, according to the first modified example, the "extended wavelength" of the Doppler sensor value obtained every period (for example, a sampling period of one second) shorter than the unit time taking the average value in time can be corrected by the "extended wavelength" of the inertial sensor value obtained every corresponding period.

Thus, in the first modified example, since the Doppler sensor value can be equivalently understood as the real-time correction, the correction accuracy of the Doppler sensor value is improved. Since the correction accuracy of the Doppler sensor value is improved, the body motion amount detection accuracy or the sleep determination accuracy is improved.

Further, since the correction of the "extended wavelength" can be illustratively realized by the correction of the amplitude change amount of the candidate Doppler sensor value added to the "extended wavelength", the calculation amount by the processor 53 can be also inhibited.

### (Second Modified Example)

In addition, the erroneous determination of the sleep determination described in Figs. 12 and 13 may be prevented or inhibited by the correction of the determination threshold value of the "body motion amount". For example, the processor 53 may correct (for example, increase) the body motion amount determination threshold value at the time corresponding to the determination timings ta to tf of Fig. 12 to a value which is not easily determined to be "awakening".

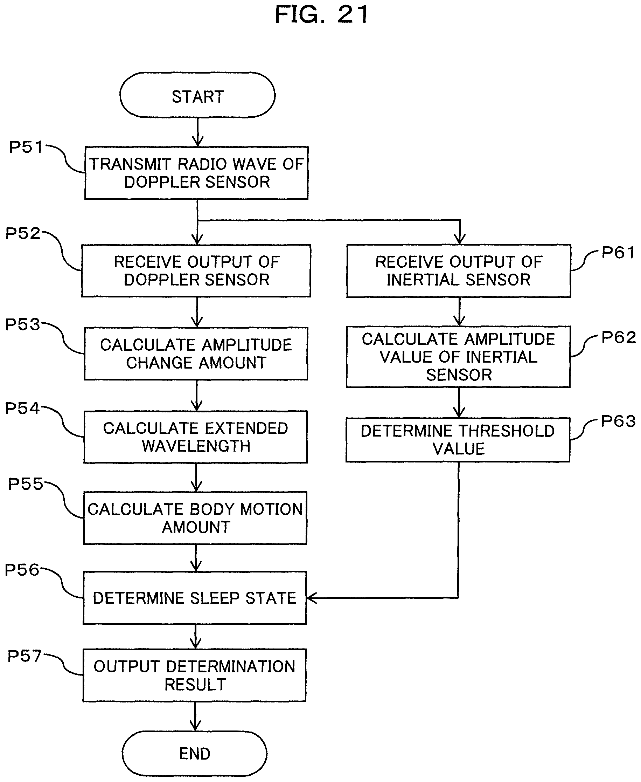

Fig. 21 illustrates an example of the sleep determination process including the correction of the determination threshold value by a flowchart. The flowchart illustrated in Fig. 21 may be understood while being performed in the processor 53 of the non-contact sleep sensor 5. The processes P51 to P54 and P55 to P57 of Fig. 21 may respectively the same as the processes P31 to P34 and P36 to P38 of the first modified example (Fig. 19). Further, the processes P61 and P62 of Fig. 21 may be respectively the same as the processes P21 and P22 of Fig. 17.

As illustrated in Fig. 21, the processor 53 transmits a radio wave from the Doppler sensor 51 to the air conditioning space during, for example, the operation of the air conditioner 2 (process P51).

In response to the transmission of the radio wave by the Doppler sensor 51, the processor 53 receives the Doppler sensor value from the Doppler sensor 51 (process P52). Further, the processor 53 receives the inertial sensor value from the inertial sensor 52 (process P61).

The processor 53 calculates the amplitude change amount based on the received Doppler sensor value as described in Fig. 16 (process P53) and calculates the "extended wavelength" based on the calculated amplitude change amount (process P54).

Further, the processor 53 calculates the "body motion amount" based on the "calculated extended wavelength" (process P55) and performs the sleep determination illustrated in Figs. 12 to 14 based on the "calculated body motion amount" (process P56). The determination threshold value used in the sleep determination may be corrected in response to the inertial sensor value.

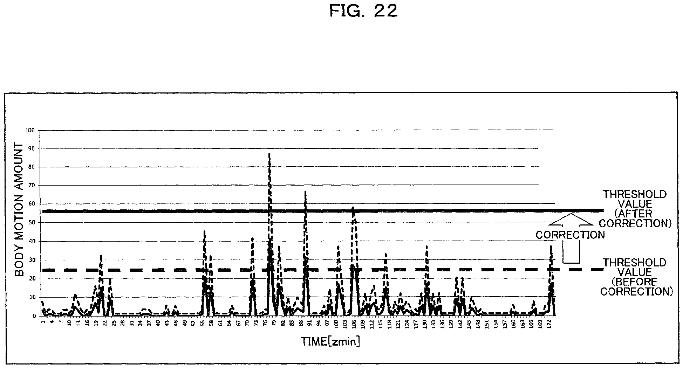

For example, the processor 53 calculates the amplitude value of the inertial sensor value received from the inertial sensor 52 (process P62) and determines the determination threshold value in response to the amplitude value (process P63). For example, as schematically illustrated in Fig. 22, the determination threshold value may be increased so that the determination based on the "body motion amount" is not easily determined to be "awakening".

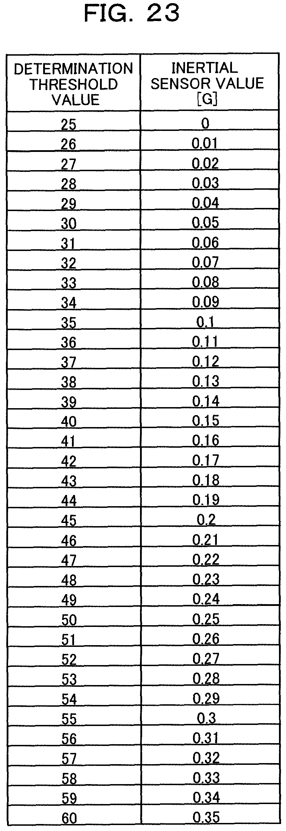

As one non-limiting example, the determination threshold value may increase by "1" whenever the inertial sensor value increases by 0.01 [G], for example, as illustrated in Fig. 23. In addition, a relation between the determination threshold value and the inertial sensor value illustrated in Fig. 23 may be illustratively stored in the memory 54.

Accordingly, in the sleep determination process P56, it is possible to avoid or inhibit the erroneous determination of the sleep determination illustrated in Figs. 12 and 13. The sleep determination result may be illustratively transmitted from the communication IF 55 to the control system 4 (process P57). In addition, the correction of the determination threshold value may be applied to the sleep determination process (P17) illustrated in Fig. 17 or the sleep determination process (P37) illustrated in Fig. 19.

As described above, according to the embodiments including the modified examples, since it is possible to cancel the vibration noise applied to the Doppler sensor value in response to the vibration during the operation of the air conditioner 2 in response to the inertial sensor value, it is possible to improve the detection accuracy of the vital information of the user in the air conditioning space.

Since it is possible to inhibit the influence of the vibration noise of the air conditioner 2 on the sleep determination based on the vital information in response to the improvement in the detection accuracy of the vital information, it is possible to improve the sleep determination accuracy.

Since the sleep determination accuracy is improved, the accuracy of the air conditioning control using the sleep determination result is improved and thus the efficiency of the air conditioning control can be improved. For example, the control system 4 can adaptively control the blowing temperature, the blowing rate, the blowing direction, and the like of the air conditioner 2 in response to the sleep determination result.

Thus, the air conditioning system 1 can provide a user with, for example, a comfortable environment that assists the user's good sleep. In addition, the air conditioning control based on the sleep determination result may be referred to as a "good sleep control" for convenience of description.

Further, as illustrated in Figs. 6 to 9, the non-contact sleep sensor 5 can be easily attached to the built air conditioner 2 (in other words, external attaching). Thus, since it is possible to realize the above-described air conditioning control regardless of the type of the air conditioner 2, it is possible to provide a comfortable environment with the user while effectively using the existing air conditioning facility.

Since the existing air conditioning facility can be effectively used, the air conditioner 2 does not need to be bought and replaced and thus the air conditioning system 1 can be decreased in cost. In the future, the sensor 5 can be built in the air conditioner 2. For example, since the air conditioning control can be performed at low cost in such a manner that the sensor 5 is attached to the built air conditioner 2 by retrofitting at the initial stage of developing the market of the air conditioning system 1, it is possible to reduce a hurdle to enter the market.

Further, in the future, there is a possibility that the air conditioning space is unable to be easily seen from the built-in position in accordance with the installation place of the air conditioner 2 even when the sensor 5 is built in the air conditioner 2. Further, there is a possibility that the air conditioning space is unable to be effectively seen from the sensor 5 due to, for example, a wall or a ceiling provided with the air conditioner 2.

Thus, when the sensor 5 is attached to the air conditioner 2 by retrofitting as described above, the visual direction or the visual range of the sensor 5 can be easily changed and adjusted. Thus, it is possible to mention that this configuration is convenient compared to the case where the sensor 5 is built in the air conditioner 2 in advance.

In addition, in the embodiment including the modified examples, a case in which the processes illustrated in Figs. 17 to 23 are performed by the processor 53 of the non-contact sleep sensor 5 has been described. However, a part or all of the processes illustrated in Figs. 17 to 23 may be performed by the control system 4 (for example, the processor 41).

Illustratively, the sensor 5 may transmit the Doppler sensor value and the inertial sensor value to the control system 4 and the control system 4 may perform the extended wavelength calculation process, the body motion amount calculation process, the correction process using the inertial sensor value, or the sleep determination process based on the received sensor values.

Alternatively, the sensor 5 may transmit the calculation value calculated in the processes to the sleep determination based on the Doppler sensor value and the inertial sensor value to the control system 4 and the control system 4 may perform the remaining processes to the sleep determination based on the received calculation values.

In a case of performing the calculation process, the correction process, or the sleep determination process by the control system 4, the function addition or the update of the control system 4 can be easily performed by modifying a program or data read by the processor 41 of the control system 4. Thus, the air conditioning system 1 can be easily updated at one time by the modification of the control system 4 instead of the modification of the sensor 5.

Further, in the embodiment including the modified examples, the sleep determination for the user in the air conditioning space has been described, but it may be determined whether the user stays in the air conditioning space based on the Doppler sensor value and the inertial sensor value. The control system 4 may remotely and adaptively control the operation of the air conditioner 2 in response to the residence and the absence of the user.

### REFERENCE NUMERALS LIST

- 1:: air conditioning system
- 2:: air conditioner
- 21:: controller
- 22:: blowing fan
- 23:: louver
- 24:: communication IF
- 25:: operation unit
- 26:: temperature sensor
- 27:: humidity sensor
- 28:: power supply circuit
- 29:: cleaning mechanism
- 30:: camera
- 3:: network (NW)
- 4:: control system
- 41:: processor
- 42:: memory
- 43:: storage device
- 44:: communication IF
- 45:: peripheral IF
- 46:: bus
- 5:: sensor (non-contact sleep sensor)
- 51:: Doppler sensor
- 511:: antenna
- 512:: local oscillator (OSC)
- 513:: Micro Control Unit (MCU)
- 514:: detection circuit
- 515:: operational amplifier (OP)
- 516:: battery
- 52:: inertial sensor
- 53:: processor
- 54:: memory
- 55:: communication IF
- 56:: power receiving IF
- 57:: bus
- 6:: router
- 7:: power cable
- 8, 9:: jig
- 200:: open/close door

## Claims

1. An air conditioner comprising:
a sensor unit that includes a Doppler sensor and an inertial sensor.

2. The air conditioner according to claim 1,
wherein the inertial sensor is configured such that an acceleration detection axis is aligned to a directivity direction of a radio wave transmitted from the Doppler sensor.

3. The air conditioner according to claim 1,
wherein the inertial sensor is operated during an operation of the Doppler sensor.

4. The air conditioner according to claim 1,
wherein the sensor unit includes a transmitter configured to transmit sensor information to a control system, and
wherein the air conditioner comprises a receiver configured to receive a control signal generated by the control system based on the sensor information.

5. The air conditioner according to any one of claims 1 to 4,
wherein the sensor unit is attached to a position receiving a vibration during an operation of the air conditioner.

6. The air conditioner according to claim 5,
wherein the sensor unit is attached to a main body of the air conditioner.

7. The air conditioner according to claim 5,
wherein the sensor unit is attached to a jigto which a main body of the air conditioner is attached.

8. The air conditioner according to c any one of claims 1 to 7,
wherein the sensor unit corrects a detected value of the Doppler sensor in response to a detected value of the inertial sensor.

9. The air conditioner according to any one of claims 1 to 7,
wherein the sensor unit corrects a threshold value, used to detect a body motion state of a user in a space corresponding to an air conditioning target of the air conditioner) based on a detected value of the Doppler sensor, based on a detected value of the inertial sensor.

10. The air conditioner according to any one of claims 1 to 7,
wherein the sensor unit obtains a length of a first waveform corresponding to a locus in which a change in detected value of the Doppler sensor is drawn per unit time in a time domain; and a length of a second waveform corresponding to a locus in which a change in detected value of the inertial sensor is drawn per unit time in a time domain and corrects the length of the first waveform in response to the length of the second waveform.

11. A sensor unit attached to an air conditioner comprising:
a Doppler sensor; and
an inertial sensor,
wherein the sensor unit is attached to a position receiving a vibration during an operation of the air conditioner.

12. The sensor unit according to claim 11,
wherein the position is a surface of a casing of the air conditioner.

13. The sensor unit according to claim 11,
wherein the position is a position contacting a jig to which a main body of the air conditioner is attached.

14. An air conditioning system comprising:
a sensor unit that includes a Doppler sensor and an inertial sensor;
an air conditioner to which the sensor unit is attached; and
a control system that is connected to the sensor unit and the air conditioner to communicate therewith, corrects a detected value of the Doppler sensor received from the sensor unit in response to a detected value of the inertial sensor received from the sensor unit, and transmits a signal generated based on a corrected value to the air conditioner.
